# FASCICULE DE BREVET EUROPEEN

(11) **EP 3 097 074 B1**
(45) Date de publication et mention de la délivrance du brevet: **18.07.2018**
(21) Numéro de dépôt: 15700694.1
(22) Date de dépôt: 19.01.2015
(51) Int. Cl.: C07C 68/00

(54) **PROCÉDÉ DE SYNTHÈSE DE CARBONATE DE DIMÉTHYLE**
VERFAHREN ZUR SYNTHESE VON DIMETHYLCARBONAT
METHOD FOR SYNTHESISING DIMETHYL CARBONATE

(30) Priorité: 22.01.2014 FR 1450506
(43) Date de publication de la demande: 30.11.2016
(73) Titulaire: Charabot SA, 06130 Grasse (FR); Institut National Polytechnique de Toulouse, 31400 Toulouse (FR); Institut National De La Recherche Agronomique, 75007 Paris (FR)
(72) Inventeur: MOULOUNGUI, Zéphirin, 31200 Toulouse (FR); MARUEJOULS, Christophe, 91280 Saint-Pierre du Perray (FR); LAVOINE-HANNEGUELLE, Sophie, 06370 Mouans-sartoux (FR)
(74) Mandataire: Cabinet BARRE LAFORGUE & associés
(86) Numéro de dépôt international: PCT/EP2015/050869
(87) Numéro de publication internationale: WO 2015/110381

(56) Documents cités:
- WU XIAOMIN ET AL: "Synthesis of dimethyl carbonate by urea alcoholysis over Zn/Al bi-functional catalysts", APPLIED CATALYSIS A: GENERAL, vol. 473, 7 janvier 2014 (2014-01-07), pages 13-20, XP028622691, ISSN: 0926-860X, DOI: 10.1016/J.APCATA.2013.12.034
- WANG H ET AL: "Highly selective synthesis of dimethyl carbonate from urea and methanol catalyzed by ionic liquids", FUEL PROCESSING TECHNOLOGY, ELSEVIER BV, NL, vol. 90, no. 10, 1 octobre 2009 (2009-10-01), pages 1198-1201, XP026611631, ISSN: 0378-3820, DOI: 10.1016/J.FUPROC.2009.05.020 [extrait le 2009-06-17]
- H. LIAN ET AL: "Processing of lignin in urea/zinc chloride deep-eutectic solvent and its use as filler in a phenol-formaldehyde resin", RSC ADVANCES, 1 janvier 2012 (2012-01-01), XP055178399, ISSN: 2046-2069, DOI: 10.1039/C4RA16734A
- ANDREW&EMSP14;P. ABBOTT ET AL: "Eutectic-Based Ionic Liquids with Metal-Containing Anions and Cations", CHEMISTRY - A EUROPEAN JOURNAL, vol. 13, no. 22, 27 juillet 2007 (2007-07-27), pages 6495-6501, XP055178320, ISSN: 0947-6539, DOI: 10.1002/chem.200601738
- SUMAROKOVA T N ET AL: "SnCl2.2(NH2)2CO and SnCl2.2(NH2)2CS", IZVESTIA AKADEMII NAUK KAZAHSKOJ SSR. SERI HIMICESKAA, NAUKA KAZSSR, ALMA-ATA, KZ, vol. 18, no. 6, 1 janvier 1968 (1968-01-01), pages 76-78, XP008172301, ISSN: 0002-3205

## Description

L'invention concerne un procédé de synthèse de carbonate de diméthyle à pression atmosphérique à partir de méthanol et d'urée. L'invention concerne aussi un procédé d'enrichissement en carbonate de diméthyle d'une composition comprenant du carbonate de diméthyle et du méthanol et un procédé de purification de carbonate de diméthyle et de préparation de carbonate de diméthyle sensiblement pur et exempt de méthanol.

On connaît déjà différents procédés permettant de synthétiser du carbonate de diméthyle. On connait (Wu Xiaomin et al., (2014), Applied Catalysis A : General, *473*, 13-20. Synthesis of dimethyl carbonate by urea alcoholysis over Zn/Al bi-functional catalysts) un procédé de synthèse de diméthyle carbonate sous pression dans un autoclave.

On connait de US 2012/0130111 un procédé de synthèse de carbonate de diméthyle à pression atmosphérique et à la température de 185°C dans lequel on introduit du méthanol à l'état gazeux dans un réacteur contenant de l'urée, ZnO à titre de catalyseur et du 2-hydroxyéthyl-N,N,N-triméthylammonium bis(trifluorométhylsulfonyl)imide (choline-NTf2) formé par mélange de chlorure de choline (chlorure de 2-hydroxyéthyl-N,N,N-triméthylammonium) et de bis(trifluorométhylsulfonyl)imide de lithium.

Un tel procédé nécessite un organe de préchauffage de méthanol liquide et sa vaporisation avant son introduction sous forme de vapeur dans le réacteur. Un tel procédé est donc complexe dans sa mise en oeuvre. Il nécessite en outre d'utiliser du 2-hydroxyéthyl-N,N,N-triméthylammonium bis(trifluorométhylsulfonyl)imide qui nécessite pour sa purification des étapes répétées de lavage à l'eau jusqu'à l'élimination complète des ions chlorures. En outre, un tel composé nécessite pour sa préparation d'utiliser le sel bis(trifluorométhylsulfonyl)imide de lithium qui est une substance toxique pour l'environnement, notamment pour les organismes aquatiques. Une telle substance est en outre toxique pour l'utilisateur par ingestion et par contact cutané.

L'invention vise à pallier les inconvénients précédemment évoqués en proposant un procédé de synthèse de carbonate de diméthyle ne nécessitant pas l'utilisation de sel bis(trifluorométhylsulfonyl)imide de lithium.

L'invention vise aussi à proposer un procédé de synthèse de carbonate de diméthyle à pression atmosphérique à partir d'urée et de méthanol permettant d'obtenir un rendement élevé de conversion de l'urée en carbonate de diméthyle sans utilisation de sel bis(trifluorométhylsulfonyl)imide de lithium et qui est donc simplifié. L'invention vise également à proposer un procédé de synthèse de carbonate de diméthyle qui est biosourcé, c'est-à-dire qui est obtenu à partir de ressources naturelles et renouvelables telles que le méthanol, l'ammoniac et le dioxyde de carbone.

L'invention vise également une composition obtenue lors de la mise en oeuvre d'un procédé de synthèse de carbonate de diméthyle selon l'invention.

L'invention vise également à atteindre ces objectifs à moindre coût, en proposant un procédé de synthèse de carbonate de diméthyle de coût de revient acceptable pour sa mise en oeuvre à l'échelle industrielle, et ne nécessitant pas de moyen spécifique de contrôle de la pression dans le réacteur de synthèse.

L'invention vise également en plus particulièrement à proposer un tel procédé de synthèse de carbonate de diméthyle qui soit compatible avec les contraintes de respect de l'environnement.

Pour ce faire, l'invention concerne un procédé de synthèse de carbonate de diméthyle à partir de méthanol et d'urée, dans lequel on utilise un milieu uréique salin comprenant au moins un sel inorganique choisi dans le groupe formé du chlorure de zinc (Zn) II, des chlorures d'étain (Sn) et du chlorure de fer (Fe) III :
caractérisé en ce que :
- on met du méthanol, en présence d'une composition catalytique, en contact avec ledit milieu uréique salin au moins partiellement liquide à une température, dite température de synthèse, supérieure à 140°C, ce par quoi des vapeurs réactionnelles sont produites ;
- on condense les vapeurs réactionnelles et on récupère un condensat desdites vapeurs réactionnelles et comprenant du carbonate de diméthyle ;
- on met en oeuvre le procédé à pression atmosphérique.

L'invention consiste donc à proposer un procédé de synthèse catalytique de carbonate de diméthyle à pression atmosphérique à partir d'urée et de méthanol selon le schéma de réaction (I) suivant : 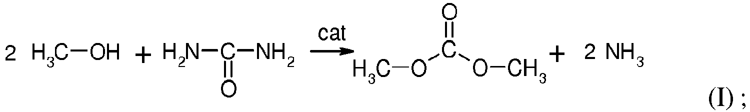 dans lequel « cat » représente un catalyseur.

Dans toute la suite ;
- le « carbonate de diméthyle » est le composé référencé au « Chemical Abstract Service » sous le numéro CAS 616-38-6 et de formule générale (II) ci-après : 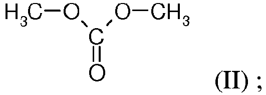
- l'expression « sel inorganique » désigne un composé électriquement neutre formé d'au moins un cation -notamment d'un cation d'un élément métallique- et d'au moins un anion, chacun des cations et des anions étant par nature inorganique, c'est-à-dire sensiblement exempts de matière organique à base d'atomes de carbone ou d'atomes de carbone associés à d'autres éléments tels que l'azote (N), l'hydrogène (H) et l'oxygène (O) ;
- l'expression « milieu uréique salin » désigne un milieu comprenant de l'urée et au moins un sel inorganique.

Dans un procédé selon l'invention, (i) on fait réagir à pression atmosphérique du méthanol liquide avec l'urée d'un milieu uréique salin au moins partiellement -notamment totalement- liquide comprenant de l'urée et au moins un sel inorganique choisi dans le groupe formé des chlorures de zinc (Zn), des chlorures d'étain (Sn) et du chlorure de fer (Fe) III (c'est-à-dire de fer de degré d'oxydation égal à 3), en présence d'au moins un catalyseur à la température de synthèse, ladite température de synthèse étant adaptée pour permettre la formation de vapeurs réactionnelles à partir du milieu réactionnel, (ii) on condense ces vapeurs réactionnelles et (iii) on récupère ce condensat comprenant du carbonate de diméthyle.

Dans un procédé selon l'invention, on forme par chauffage un milieu uréique salin au moins partiellement liquide formé à partir d'urée et d'au moins un sel inorganique choisi dans le groupe formé du chlorure de zinc (Zn) II, des chlorures d'étain (Sn) -notamment du chlorure d'étain (Sn) II- et du chlorure de fer (Fe) III et on met du méthanol en contact de ce milieu uréique salin au moins partiellement liquide. Le milieu uréique salin présente donc une phase liquide à la température de réaction. Il est cependant possible qu'il présente aussi une phase solide.

Les inventeurs ont déterminé que l'utilisation d'un tel milieu uréique salin comprenant au moins un tel sel inorganique et de l'urée est apte à retenir au moins une partie du méthanol dans le milieu uréique salin, et à permettre sa réaction avec l'urée en présence du catalyseur pour former du carbonate de diméthyle.

Les inventeurs ont aussi constaté que le milieu uréique salin permet de retenir au moins une partie du carbamate de méthyle qui est un intermédiaire de synthèse formé à partir de l'urée et du méthanol et permet donc sa réaction avec le méthanol pour former le carbonate de diméthyle. Le milieu uréique salin permet donc de retenir l'intermédiaire de synthèse et de limiter-voire d'éviter-son entrainement sous forme de vapeurs. Il se forme ainsi un condensat comprenant du carbonate de diméthyle et qui est sensiblement exempt de carbamate de méthyle. Une étape finale de séparation du carbonate de diméthyle et du carbamate de méthyle est donc inutile.

Dans un procédé selon l'invention, on forme un milieu réactionnel comprenant le milieu uréique salin apte à retenir du méthanol à une température supérieure à 140°C et le catalyseur, on forme du carbamate de méthyle et du carbonate de diméthyle et on extrait le carbonate de diméthyle formé hors du milieu réactionnel de façon à déplacer l'équilibre de la réaction dans le sens de la consommation de l'urée, du méthanol et du carbamate de méthyle, et dans le sens de la formation de diméthyle carbonate.

En outre, les inventeurs ont constaté que le milieu uréique salin permet de moduler l'élimination/rétention de l'ammoniac à l'état gazeux libéré lors de la réaction du méthanol et de l'urée (ou du carbamate de méthyle). Une telle élimination/rétention permet de moduler les paramètres cinétiques et l'équilibre de la réaction.

Ainsi, un procédé selon l'invention permet de retenir les réactifs (méthanol, urée) et l'intermédiaire de synthèse (carbamate de méthyle) dans le milieu uréique salin à la température de synthèse mais permet aussi d'extraire le produit de la réaction (carbonate de diméthyle) par condensation des vapeurs du milieu de réaction.

Avantageusement et selon l'invention, on choisit chaque sel du milieu uréique salin dans le groupe formé de SnCℓ₂, de FeCℓ₃ et de ZnCℓ₂ et de leurs mélanges. Ainsi, le milieu uréique salin est exclusivement formé d'au moins un sel inorganique et d'urée.

Avantageusement et selon l'invention, la température de synthèse est supérieure à de l'ordre de 140°C.

Avantageusement et selon l'invention, la température de synthèse est comprise entre de l'ordre de 140°C et de l'ordre de 160°C.

Avantageusement et selon l'invention, lors d'une première étape opératoire, on forme un milieu réactionnel comprenant le milieu uréique salin, la composition catalytique et une première quantité de méthanol et on maintient le milieu réactionnel à la température de synthèse, le condensat formé étant réintroduit dans le milieu réactionnel, puis lors d'une deuxième étape opératoire subséquente, on introduit un flux de méthanol dans le milieu réactionnel et on prélève un flux du condensat des vapeurs du milieu réactionnel. Dans un procédé selon l'invention, on réalise la première étape opératoire en réintroduisant le condensat dans le milieu réactionnel, c'est-à-dire sans prélèvement dudit condensat. Lors de la deuxième étape opératoire subséquente, on prélève le condensat et on introduit simultanément audit prélèvement un flux de méthanol dans le milieu réactionnel. Avantageusement et selon l'invention, on réalise la première étape opératoire dans des conditions, dites « à taux de reflux infini », telles que le rapport entre le volume de liquide condensé (reflux) et réintroduit dans le milieu réactionnel et le volume de liquide condensé (condensat) et séparé du milieu réactionnel est infini, c'est-à-dire que la totalité du condensat est réintroduite dans le milieu réactionnel.

Avantageusement, toutes les étapes du procédé selon l'invention sont réalisées à pression atmosphérique.

Avantageusement et selon l'invention, on réalise la première étape opératoire dans un récipient à pression atmosphérique et la deuxième étape opératoire dans le même récipient à pression atmosphérique.

Avantageusement et selon l'invention, la première étape opératoire est d'une durée de l'ordre de 15 min. On maintient donc le milieu réactionnel à taux de reflux infini pendant une durée de l'ordre de 15 min.

Avantageusement et selon une première variante d'un procédé conforme à l'invention, on ajoute le méthanol en flux continu dans le milieu réactionnel. Selon cette première variante, le flux de méthanol ajouté au milieu réactionnel est donc un flux ininterrompu pendant toute la durée de la réaction. Rien n'exclut cependant de faire varier la valeur de ce flux, ladite valeur de flux étant supérieure ou égale à une valeur de flux minimale non nulle.

Avantageusement, on ajoute le méthanol dans le milieu réactionnel en flux continu et on récupère le condensat comprenant du carbonate de diméthyle en continu lors de la réaction. On adapte le débit du flux de méthanol introduit dans le milieu réactionnel et la température de chauffage de façon à former par synthèse le carbonate de diméthyle, à collecter des vapeurs réactionnelles et récupérer le condensat comprenant le carbonate de diméthyle.

Avantageusement et selon une deuxième variante d'un procédé selon l'invention, on ajoute le méthanol en flux discontinu dans le milieu réactionnel. Selon cette deuxième variante, on procède par additions successives de quantités déterminées (discrètes) de méthanol dans le milieu réactionnel. On adapte la fréquence d'addition et le volume de ces quantités prédéterminées de méthanol de façon à former du carbonate de diméthyle dans le milieu réactionnel, son évaporation et sa condensation pour former le condensat.

Avantageusement, on ajoute le méthanol dans le milieu réactionnel en flux discontinu et on récupère le condensat comprenant du carbonate de diméthyle en continu lors de la réaction.

En variante, on ajoute le méthanol dans le milieu réactionnel en flux discontinu et on récupère le condensat comprenant du carbonate de diméthyle par prélèvements successifs. On prélève donc le condensat comprenant du carbonate de diméthyle de façon discontinue. On réalise donc une pluralité d'ajouts discrets successifs de méthanol dans le milieu réactionnel et on récupère le condensat comprenant du carbonate de diméthyle par prélèvements successifs.

Avantageusement et selon l'invention, on forme initialement le milieu uréique salin par chauffage d'une composition saline comprenant chaque sel inorganique et l'urée. Dans un procédé selon l'invention, on forme donc un milieu uréique salin à l'état au moins partiellement liquide par chauffage de la composition saline.

Rien n'exclut cependant de former le milieu uréique salin directement par chauffage d'une composition saline formée uniquement de chaque sel inorganique et de l'urée. Le milieu uréique salin est ainsi constitué uniquement de chaque sel inorganique et d'urée.

Avantageusement et selon l'invention, on chauffe la composition saline à une température comprise entre 80 °C et 125 °C -notamment de l'ordre de 100°C- et pendant une durée d'au moins 10 min -notamment comprise entre 10 minutes et 3 heures-. On chauffe donc la composition saline à une température suffisante pour former le milieu uréique salin à l'état au moins partiellement liquide.

Avantageusement, dans un procédé selon l'invention :
- on forme d'abord le milieu uréique salin par chauffage de chaque sel inorganique et de l'urée à une température comprise entre 80°C et 125°C pendant une durée comprise entre 10 min et 3 heures, puis ;
- on ajoute la composition catalytique au milieu uréique salin formé, puis ;
- lors de la première étape opératoire, on ajoute une première quantité de méthanol, puis on porte le mélange obtenu à une température supérieure à 140°C -notamment comprise entre de l'ordre de 140°C et de l'ordre de 160°C- et on maintient cette température pendant 15 minutes sans addition supplémentaire de méthanol ni collecte de condensat formé à partir des vapeurs réactionnelles, puis ;
- lors de la deuxième étape opératoire, on ajoute un flux de méthanol et on collecte les vapeurs réactionnelles.

Avantageusement et selon l'invention, on prépare le milieu uréique salin avec une quantité initiale d'urée et une quantité initiale de chaque sel inorganique dans un rapport massique de chaque sel inorganique par rapport à l'urée compris entre 2 et 10.

Avantageusement et selon l'invention, on prépare le milieu uréique salin avec une quantité initiale d'urée et une quantité initiale de sels inorganiques, les sels inorganiques et l'urée étant présents dans le milieu uréique dans un rapport massique des sels inorganiques par rapport à l'urée initiale compris entre 4 et 10.

Rien n'exclut de réaliser le milieu uréique salin par chauffage du mélange formé de chaque sel inorganique, de l'urée, du catalyseur et d'une première quantité de méthanol à la température de réaction sans prélèvement du condensat des vapeurs réactionnelles, puis d'ajouter le flux de méthanol en prélevant le condensat des vapeurs réactionnelles. En variante, il est possible de préparer le milieu uréique salin par chauffage d'urée et de un ou d'une pluralité de sels inorganiques puis d'ajouter une quantité de la composition catalytique dans le méthanol.

Avantageusement et selon l'invention, le milieu uréique salin est un milieu conducteur -notamment électro-conducteur et thermo-conducteur-.

Avantageusement et selon l'invention, la composition catalytique comprend au moins un composé choisi dans le groupe formé de l'oxyde de zinc et du sulfate de zinc.

Avantageusement, en variante ou en combinaison, la composition catalytique comprend un oxyde de zinc. Avantageusement, en variante ou en combinaison, la composition catalytique est formée d'oxyde de zinc. Avantageusement et selon l'invention, la composition catalytique comprend aussi du sulfate de zinc. Avantageusement et selon l'invention, la composition catalytique comprend de l'oxyde de zinc et du sulfate de zinc -notamment en proportions massiques sensiblement égales-.

Avantageusement et selon l'invention, la composition catalytique est constituée (c'est-à-dire formée uniquement) d'oxyde de zinc et de sulfate de zinc -notamment en proportions massiques sensiblement égales-.

Avantageusement et selon l'invention, la composition catalytique est à l'état solide. Avantageusement, il est donc possible de recycler ladite composition catalytique solide pour une utilisation ultérieure.

Avantageusement et selon l'invention, on prépare la composition catalytique par calcination d'un mélange solide comprenant de l'oxyde de zinc et du sulfate de zinc à une température comprise entre 300°C et 700°C. Avantageusement et selon l'invention, on prépare la composition catalytique par calcination d'un mélange solide formé uniquement d'oxyde de zinc et de sulfate de zinc à une température comprise entre 300°C et 700°C.

Avantageusement et selon l'invention, on ajoute le méthanol à l'état liquide dans le milieu réactionnel. Dans un procédé de synthèse de carbonate de diméthyle selon l'invention, on introduit du méthanol à l'état liquide dans un réacteur chauffé à la pression atmosphérique et contenant le milieu uréique salin, on forme des vapeurs réactionnelles comprenant du carbonate de diméthyle et on récupère le condensat desdites vapeurs réactionnelles.

Avantageusement, dans un mode de réalisation particulier selon l'invention, on introduit un flux d'au moins une partie -notamment de la totalité- du condensat des vapeurs réactionnelles dans un milieu réactionnel maintenu à la température de synthèse et comprenant une quantité de milieu uréique salin et une quantité de composition catalytique, ce par quoi des vapeurs réactionnelles sont produites, on condense lesdites vapeurs réactionnelles et on récupère un condensat desdites vapeurs réactionnelles comprenant du carbonate de diméthyle. Avantageusement, dans ce mode de réalisation de l'invention, on réalise une étape de recyclage d'au moins une partie -notamment de la totalité- du condensat des vapeurs réactionnelles comprenant du méthanol et du carbonate de diméthyle.

Avantageusement et selon l'invention, on soumet au moins une partie du condensat -issu d'une première synthèse de carbonate de diméthyle ou d'une étape de recyclage- à une première étape de distillation fractionnée de séparation de méthanol et d'un mélange azéotropique de carbonate de diméthyle et de méthanol. Avantageusement, le mélange azéotropique comprend une proportion massique de carbonate de diméthyle de l'ordre de 30 % dans le méthanol.

Avantageusement et selon l'invention, on réalise une deuxième étape de distillation fractionnée dans laquelle on met au moins une partie du mélange azéotropique en contact avec au moins un sel inorganique et on chauffe le mélange formé de façon à former par distillation/condensation à pression atmosphérique un condensat enrichi en carbonate de diméthyle et comprenant au moins 35 % en masse de carbonate de diméthyle. Dans cette variante d'un procédé selon l'invention, on enrichit le mélange azéotropique par distillation/condensation dudit mélange azéotropique placé en contact avec au moins un sel inorganique choisi dans le groupe formé du chlorure de zinc (Zn) II (ZnCℓ₂), des chlorures d'étain (Sn) -notamment du chlorure d'étain (Sn) II (SnCℓ₂)- et du chlorure de fer (Fe) III (FeCℓ₃). Dans une telle étape d'enrichissement du mélange azéotropique en présence d'au moins un tel sel inorganique, on piège au moins une partie du méthanol du mélange azéotropique dans le milieu de distillation et on forme le condensat enrichi en carbonate de diméthyle.

Dans cette variante d'un procédé selon l'invention, on réalise la deuxième étape de distillation fractionnée du mélange azéotrope en absence d'urée.

Avantageusement, dans une autre variante d'un procédé selon l'invention, on soumet le condensat enrichi à une distillation à pression atmosphérique adaptée pour pouvoir former un condensat formé d'un mélange azéotrope de carbonate de diméthyle et de méthanol et un raffinat formé de carbonate de diméthyle. Avantageusement, le raffinat est formé de carbonate de diméthyle sensiblement pur. Dans cette variante d'un procédé selon l'invention, on récupère dans le réacteur de distillation le raffinat formé de carbonate de diméthyle sensiblement pur.

L'invention s'étend par ailleurs à toutes les utilisations à pression atmosphérique d'un milieu uréique salin à l'état au moins partiellement liquide à une température supérieure à 140°C et comprenant de l'urée et au moins un sel inorganique choisi dans le groupe formé du chlorure de zinc (Zn) II, des chlorures d'étain (Sn) et du chlorure de fer (Fe) III -notamment SnCℓ₂, FeCℓ₃ et ZnCℓ₂ à différents taux d'hydratation-.

L'invention s'étend en particulier à toutes les utilisations d'un tel milieu uréique salin en synthèse organique, c'est-à-dire dans un procédé de synthèse et en particulier dans un procédé de synthèse et de fabrication de carbonate de diméthyle.

L'invention concerne également un procédé de synthèse de carbonate de diméthyle à pression atmosphérique, un milieu uréique salin et l'utilisation d'un tel milieu uréique salin caractérisés en combinaison par tout ou partie des caractéristiques mentionnées ci-dessus ou ci-après.

D'autres buts, caractéristiques et avantages de l'invention apparaîtront à la lecture de la description suivante de différents modes de réalisation d'un procédé selon l'invention, des exemples donnés uniquement à titre non limitatif et de la figure unique illustrant les étapes d'une variante particulière d'un procédé selon l'invention.

Dans un procédé de synthèse de carbonate de diméthyle selon l'invention, on utilise un dispositif 1 (expérimental ou industriel) de synthèse à pression atmosphérique comprenant un réacteur à double enveloppe couplé à une colonne de distillation permettant le fonctionnement du dispositif expérimental à taux de reflux infini. La colonne de distillation comprend un condenseur et est équipée à la base dudit condenseur d'un organe de contrôle du reflux permettant, dans une première position fermée de l'organe de contrôle de reflux, de guider le condensat vers le réacteur de synthèse via la colonne de distillation (taux de reflux infini), et dans une deuxième position ouverte de l'organe de contrôle de reflux, de guider le condensat vers un récipient de collecte distinct du réacteur de synthèse dans lequel est collecté en continu le condensat comprenant le carbonate de diméthyle.

Le réacteur de synthèse est en outre équipé d'un organe de pompage et d'introduction de méthanol liquide dans le réacteur de chauffage à double enveloppe, de préférence configuré pour permettre une introduction du méthanol liquide goutte à goutte par le ciel du réacteur et de façon à pouvoir mettre en contact le méthanol liquide et le milieu liquide/solide de réaction dès l'introduction du méthanol. Le dispositif expérimental est équipé d'un organe d'agitation mécanique du contenu du réacteur à double enveloppe

Dans un premier mode de réalisation d'un procédé de synthèse de carbonate de diméthyle selon l'invention, on place une quantité d'au moins un sel inorganique -notamment un chlorure d'un élément métallique- solide et éventuellement de l'urée dans un réacteur de synthèse d'un dispositif de distillation à pression atmosphérique comprenant une colonne de distillation surmontant le réacteur de synthèse, de façon à former par chauffage à une température comprise entre 80°C et 125°C pendant 10 min à 90 min un milieu uréique salin au moins partiellement liquide. Un tel milieu uréique salin est un milieu conducteur et qui présente en outre la propriété de retenir le méthanol, notamment le méthanol liquide. En particulier, on choisit chaque sel métallique dans le groupe formé du chlorure de zinc (ZnCℓ₂), du chlorure d'étain (SnCℓ₂) et du chlorure de fer (FeCℓ₃). On choisit plus particulièrement le chlorure d'étain (SnCℓ₂) qui tout en présentant des propriétés rétentrices du méthanol, ne retient pas l'ammoniac formé lors de la formation du carbonate de diméthyle et qui peut donc être piégé dans un piège à eau.

Le rapport de la quantité molaire de sels inorganiques sur la quantité molaire d'urée est en général compris entre 1 et 10, notamment de l'ordre de 3.

On ajoute ensuite dans le réacteur de synthèse une quantité de catalyseur solide, par exemple formé d'oxyde de zinc et de sulfate de zinc en proportions massiques sensiblement égales et calciné préalablement à une température comprise entre 300°C et 700°C et conservé à l'abri de l'humidité. Le rapport de la quantité molaire d'urée sur la quantité molaire de catalyseur solide est en général compris entre 10 et 100, notamment de l'ordre de 50.

Il est possible, à cette étape, d'ajouter dans le réacteur de synthèse à pression atmosphérique une quantité de méthanol liquide avant de porter la température dans le réacteur de synthèse à une température de synthèse supérieure à 140°C -notamment comprise entre de l'ordre de 140°C et de l'ordre de 160°C-. On adapte la température de synthèse dans le réacteur de synthèse de façon à maintenir le milieu uréique salin contenant, le cas échéant, du méthanol dans un régime de reflux infini pendant de l'ordre de 15 min.

On maintient la température dans le réacteur de synthèse de façon à former des vapeurs réactionnelles et on procède à une addition de méthanol liquide en prélevant simultanément à cette addition un condensat formé par condensation des vapeurs réactionnelles produite dans le réacteur de synthèse et comprenant du carbonate de diméthyle. Dès lors, le dispositif de distillation à pression atmosphérique ne fonctionne plus à taux de reflux infini. Le condensat formé par évaporation/condensation du milieu réactionnel est constitué de méthanol liquide et de carbonate de diméthyle dans une proportion massique de l'ordre de 5 % de carbonate de diméthyle.

Ainsi, on extrait du milieu uréique salin au moins une partie du carbonate de diméthyle formé au contact du milieu uréique salin et on déplace l'équilibre de la réaction vers la formation dudit carbonate de diméthyle. En outre, lors de la réaction du méthanol et de l'urée en présence du(des) sel(s) inorganiques et du catalyseur, il se produit un dégagement d'ammoniac sous forme gazeuse qui est éliminé, au moins partiellement, du milieu réactionnel. Les inventeurs ont observé que, de façon imprévisible et surprenante, le(les) sel(s) inorganique(s) du milieu liquide/solide, éventuellement en combinaison avec l'urée et/ou le(s) catalyseur(s), permettent en même temps de retenir dans le milieu uréique salin une quantité de méthanol à l'état liquide pour sa réaction avec l'urée et de ne pas retenir dans le milieu uréique salin l'ammoniac dégagé lors de ladite réaction.

Il est possible d'ajouter la quantité de méthanol liquide en flux continu dans le réacteur de synthèse. Bien entendu, il est possible de réaliser un tel ajout en flux continu en utilisant une pompe d'introduction de méthanol liquide dont le débit est contrôlé. Le débit d'introduction du méthanol liquide dans le milieu uréique salin peut être compris entre 10 mL/h et 150 mL/h selon les quantités initiales de sel(s) inorganique(s), d'urée et de catalyseur solide. Rien n'empêche cependant d'ajouter le méthanol liquide de façon discontinue par des apports successifs de volumes discrets dudit méthanol liquide dans le milieu uréique salin.

Le volume total de méthanol liquide ajouté peut varier selon les conditions opératoires. Bien évidemment, l'addition dans le réacteur de synthèse d'un volume important de méthanol liquide pourra permettre d'obtenir un taux de conversion élevé de l'urée en carbonate de diméthyle. Cependant, dans de telles conditions, la valeur du taux de conversion de l'urée initiale en carbonate de diméthyle (%) rapporté à la concentration (mole/L) d'urée initiale et à la concentration de méthanol liquide ajouté sera minimisée.

On interrompt la réaction de synthèse du carbonate de diméthyle en interrompant l'introduction du méthanol liquide dans le milieu réactionnel tout en maintenant le chauffage du réacteur à la température de synthèse pendant de l'ordre de 15 min après l'arrêt de l'introduction du méthanol liquide et tout en collectant le condensat. On interrompt ensuite le chauffage et on laisse le réacteur de synthèse et le milieu réactionnel refroidir jusqu'à la température ambiante.

Dans un deuxième mode de réalisation d'un procédé de synthèse de carbonate de diméthyle selon l'invention, on prépare dans le réacteur de synthèse un milieu uréique salin par mélange d'au moins un sel inorganique, d'urée et d'un catalyseur solide et, le cas échéant de méthanol liquide. On chauffe le réacteur à pression atmosphérique et à une température comprise entre de l'ordre de 140°C et de l'ordre de 160°C de façon à former le milieu uréique salin et en le maintenant à un taux de reflux infini pendant une durée de l'ordre de 15 min. Dès lors que le milieu uréique salin est à reflux infini, on ajoute du méthanol liquide et on collecte le condensat formé de méthanol et de carbonate de diméthyle.

Dans un troisième mode de réalisation d'un procédé de synthèse selon l'invention, on collecte le condensat formé de méthanol et de carbonate de diméthyle et on soumet ledit condensat à une étape subséquente d'enrichissement du condensat en carbonate de diméthyle par distillation fractionnée lors de laquelle on collecte une première fraction, formée de méthanol et susceptible d'être recyclée dans une étape ultérieure de synthèse de carbonate de diméthyle, puis une deuxième fraction, dite mélange azéotropique, formée de méthanol et de carbonate de diméthyle. Dans ce troisième mode de réalisation, on forme donc un mélange proche de l'azéotrope et enrichi en carbonate de diméthyle par rapport au condensat et comprenant jusqu'à 30 % (en masse) de carbonate de diméthyle et au moins 70 % en masse de méthanol. Dans ce troisième mode de réalisation, on collecte la première fraction riche en méthanol et on recycle ladite première fraction riche en méthanol dans un procédé de synthèse de carbonate de diméthyle à pression atmosphérique selon l'un des trois premiers modes de réalisation d'un procédé selon l'invention. Il est aussi possible de réutiliser la première fraction riche en méthanol dans tout procédé dans lequel du méthanol peut être utilisé.

Dans un quatrième mode de réalisation d'un procédé de synthèse selon l'invention, on collecte le mélange azéotropique et on le soumet à une deuxième étape subséquente de distillation fractionnée dans laquelle on place le mélange azéotropique au contact de sel(s) inorganique(s) dans le réacteur de synthèse d'un dispositif de distillation à pression atmosphérique comprenant une colonne de distillation surmontant le réacteur de synthèse, on chauffe le mélange azéotropique au contact de sel(s) inorganique(s) de façon à piéger au moins une partie du méthanol du mélange azéotropique dans le réacteur de synthèse et former, par distillation/condensation à pression atmosphérique, une composition de carbonate de diméthyle comprenant de l'ordre de 35 % en masse de carbonate de diméthyle.

Dans ce quatrième mode de réalisation d'un procédé de synthèse selon l'invention, on forme donc dans le réacteur de synthèse une composition comprenant du méthanol, des sels inorganiques, éventuellement du carbonate de diméthyle. Les sels inorganiques sont susceptibles de pouvoir être précipités pour une réutilisation ultérieure dans le réacteur de synthèse d'un procédé selon l'un des trois premiers modes de réalisation ou d'une étape subséquente de résolution azéotropique selon le quatrième mode de réalisation.

Dans un cinquième mode de réalisation d'un procédé de synthèse selon l'invention, on soumet la composition de carbonate de diméthyle comprenant de l'ordre de 35 % en masse de carbonate de diméthyle à une étape de distillation fractionnée à pression atmosphérique adaptée pour pouvoir former un condensat formé d'un mélange azéotropique de carbonate de diméthyle et de méthanol et un raffinat formé de carbonate de diméthyle sensiblement pur.

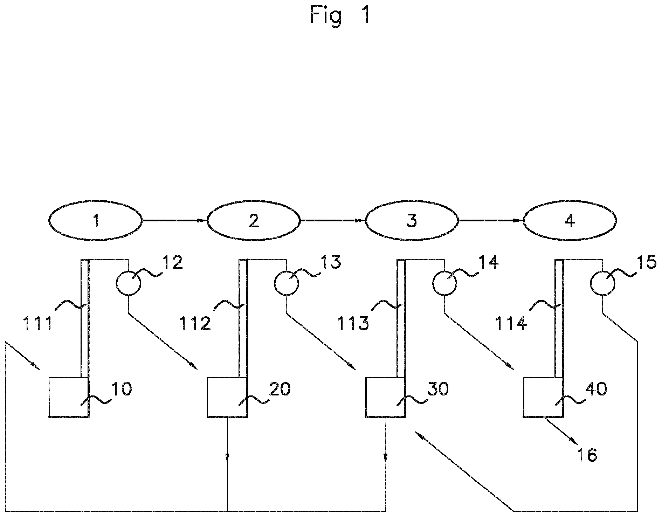

Un procédé dans lequel sont combinés le premier ou le deuxième mode de réalisation avec les troisième, quatrième et cinquième modes de réalisation est représenté en figure 1. Dans une première étape 1 de synthèse du procédé représenté, on forme dans le réacteur 10 de synthèse des vapeurs réactionnelles par chauffage de méthanol, d'un milieu uréique salin et d'un catalyseur. On condense ces vapeurs dans une colonne 111 de distillation et on collecte un distillat en tête 12 de la colonne de distillation. Le distillat comprend une proportion de l'ordre de 5 % de carbonate de diméthyle et du méthanol.

Dans une deuxième étape 2 d'enrichissement du distillat en carbonate de diméthyle, on place le distillat formé à la première étape 1 dans un réacteur 20 de distillation et on chauffe de façon à prélever par distillation en tête d'une colonne 112 de distillation, une composition 13 azéotropique comprenant de l'ordre de 30 % de carbonate de diméthyle et conserver dans le réacteur 20 l'excès de méthanol qui est susceptible d'être avantageusement réutiliser pour une étape de synthèse 1 ultérieure.

Dans une troisième étape 3 de séparation, on place la composition 13 azéotropique dans un réacteur 30 de chauffage en présence d'une quantité de sel d'un milieu uréique salin et on chauffe le réacteur 30 de façon à former en tête d'une colonne 113 de distillation une composition 14 enrichie en carbonate de diméthyle et comprenant par exemple de l'ordre de 35 % de carbonate de diméthyle et du méthanol. On récupère aussi dans le réacteur 30 un mélange comprenant du méthanol, du carbonate de diméthyle et du sel du milieu uréique salin. Ce mélange est susceptible de pouvoir être avantageusement réutilisé dans un réacteur 10 de synthèse d'une étape 1 de synthèse ultérieure.

Dans une quatrième étape 4 de purification du carbonate de diméthyle, on soumet la composition 14 enrichie à une distillation dans un réacteur 40 de distillation et dans laquelle on forme en tête d'une colonne 114 de distillation une composition 15 azéotropique comprenant de l'ordre de 30 % de carbonate de diméthyle et dans le réacteur 40 de distillation du carbonate de diméthyle 16 sensiblement pur. Dans un procédé selon l'invention, on recycle avantageusement la composition 15 azéotropique dans le réacteur 30 de chauffage de façon à former une composition 14 enrichie subséquente.
EXEMPLE 1 - Préparation du catalyseur oxyde de zinc/sulfate de zinc.
   Dans un récipient, on mélange des masses équivalentes d'oxyde de zinc et de sulfate de zinc). Après broyage, on dilue dans du dichlorométhane pour homogénéiser le mélange. Après évaporation du solvant, on calcine la préparation pendant 4h à 500°C. Le catalyseur est ensuite conservé à l'abri de l'humidité dans un récipient hermétiquement clos.
EXEMPLE 2 - Synthèse de carbonate de diméthyle à partir d'urée, de méthanol, de chlorure d'étain (SnCℓ₂) et d'oxyde de zinc/sulfate de zinc (ZnO/ZnSO₄) à titre de catalyseur.
   Dans un réacteur de 250 mL à double enveloppe équipé d'une colonne de distillation et d'un dispositif d'agitation, on introduit 75 g de SnCℓ₂ et 8 g d'urée. On chauffe sous agitation le mélange obtenu en portant le fluide circulant dans la double enveloppe à la température de 100°C pendant 20 min de façon à former un milieu uréique salin. On ajoute ensuite dans le milieu uréique salin obtenu 0,4 g de catalyseur ZnO/ZnSO₄ tel qu'obtenu à l'exemple 1. On ajoute ensuite 15 mL de méthanol et on porte la température de la double enveloppe à 165°C de façon à atteindre la température de synthèse dans le réacteur (compte tenu de l'addition de méthanol froid, de l'évaporation des constituants volatils) en maintenant des conditions de reflux infini pendant 15 min. Aucun condensat n'est donc prélevé à cette étape. On introduit alors dans le réacteur du méthanol liquide à raison de 120 mL de méthanol par heure (120 mL/h) pendant 4 heures (soit un total de 480 mL de méthanol). Durant cette phase, on prélève en continu un condensat en tête de la colonne de distillation. Après 4h de réaction, on interrompt l'introduction du méthanol puis, après 15 min, le chauffage du réacteur.
   L'analyse par chromatographie en phase gazeuse du condensat permet de déterminer que le taux de conversion de l'urée en carbonate de diméthyle est de 33 %. La concentration du carbonate de diméthyle dans le distillat est de 11,4 mg de carbonate de diméthyle par gramme de condensat.
EXEMPLE 3 - Synthèse de carbonate de diméthyle à partir d'urée, de méthanol, de chlorure de zinc (ZnCℓ₂) et d'oxyde de zinc/sulfate de zinc (ZnO/ZnSO₄) à titre de catalyseur.
   Dans un réacteur de 250 mL à double enveloppe équipé d'une colonne de distillation et d'un dispositif d'agitation, on introduit 75 g de ZnCℓ₂ et 8 g d'urée. On chauffe sous agitation le mélange obtenu en portant le fluide circulant dans la double enveloppe à la température de 100°C pendant 20 min de façon à former un milieu uréique salin liquide. On ajoute ensuite dans le milieu uréique salin obtenu 0,4 g de catalyseur ZnO/ZnSO₄ tel qu'obtenu à l'exemple 1. On ajoute ensuite 35 mL de méthanol et on porte la température de la double enveloppe à 165°C de façon à atteindre la température de synthèse dans le réacteur en maintenant des conditions de reflux infini pendant 15 min. Aucun condensat n'est donc prélevé à cette étape. On introduit alors dans le réacteur du méthanol liquide à raison de 115 mL de méthanol par heure (115 mL/h) pendant 4 heures (soit un total de 480 mL de méthanol). Durant cette phase, on prélève en continu un condensat en tête de la colonne de distillation. Après 4h de réaction, on interrompt l'introduction du méthanol puis, après 15 min, le chauffage du réacteur.
   L'analyse par chromatographie en phase gazeuse du condensat permet de déterminer que le taux de conversion de l'urée en carbonate de diméthyle est de 23 %. La concentration du carbonate de diméthyle dans le distillat est de 8,5 mg de carbonate de diméthyle par gramme de condensat.
EXEMPLE 4 - Synthèse de carbonate de diméthyle à partir d'urée, de méthanol, de chlorure de zinc (ZnCℓ₂) et d'oxyde de zinc/sulfate de zinc (ZnO/ZnSO₄) à titre de catalyseur.
   Dans un réacteur de 250 mL à double enveloppe équipé d'une colonne de distillation et d'un dispositif d'agitation, on introduit 75 g de ZnCℓ₂, 8 g d'urée et 0,4 g de catalyseur ZnO/ZnSO₄ tel qu'obtenu à l'exemple 1. On ajoute 15 mL de méthanol et on porte la température du fluide circulant dans la double enveloppe à la température de 165°C de façon à atteindre la température de synthèse dans le réacteur en maintenant des conditions de reflux infini pendant 15 min. On introduit ensuite dans le réacteur du méthanol liquide en flux continu à raison de 120 mL de méthanol par heure (120 mL/h) pendant 4 heures (soit un total de 480 mL de méthanol). Durant cette phase d'addition de méthanol, on prélève en continu un condensat en tête de la colonne de distillation. Après 4h de réaction, on interrompt l'introduction du méthanol puis, après 15 min, le chauffage du réacteur.
   L'analyse par chromatographie en phase gazeuse du condensat permet de déterminer que le taux de conversion de l'urée en carbonate de diméthyle est de 29 %. La concentration du carbonate de diméthyle dans le distillat est de 10,2 mg de carbonate de diméthyle par gramme de condensat.
EXEMPLE 5 - Synthèse de carbonate de diméthyle à partir d'urée, de méthanol, d'oxyde de zinc et de sulfate de zinc à titre de catalyseur et de chlorure de zinc à titre de sel inorganique.
   Dans un ballon de 250 mL équipé d'une colonne de distillation surmontée d'un appareil de Dean-Stark, on introduit 20 g de ZnCℓ₂ et 5 g d'urée puis on chauffe le mélange obtenu dans un bain d'huile à la température de 100°C pendant 2 heures à la pression atmosphérique. On ajoute ensuite 0,1 g de catalyseur ZnO/ZnSO₄ tel que décrit à l'exemple 1. On ajoute alors 5 g de méthanol et on porte la température du bain d'huile à 165°C de façon à atteindre la température de synthèse dans le ballon et on maintient cette température pendant 15 min. Toujours à la température de synthèse, on réalise une addition de méthanol à raison de 5 g de méthanol par heure pendant 4 heures en prélevant le condensat en continu. Après 4h de réaction, on interrompt l'introduction du méthanol puis, après 15 min supplémentaires, on retire le ballon du bain d'huile.
   Le taux de conversion de l'urée en carbonate de diméthyle est de 16 %.
EXEMPLE 6 - Synthèse de carbonate de diméthyle à partir d'urée, de méthanol, de ZnO/ZnSO₄ à titre de catalyseur et de SnCℓ₂ à titre de sel inorganique.
   On opère de façon identique à l'exemple 5 mais en remplaçant ZnCℓ₂ par SnCℓ₂. Le taux de conversion de l'urée en carbonate de diméthyle est de 33 %.
EXEMPLE 7 - Synthèse de carbonate de diméthyle à partir d'urée, de méthanol, de ZnO/ZnSO₄ à titre de catalyseur et de FeCℓ₃ à titre de sel inorganique.
   On opère de façon identique à l'exemple 5 mais en remplaçant ZnCℓ₂ par FeCℓ₃. Le taux de conversion de l'urée en carbonate de diméthyle est de 30%.
EXEMPLE 8 - Synthèse de carbonate de diméthyle à partir d'urée, de méthanol, de ZnO/ZnSO₄ à titre de catalyseur et d'un mélange SnCl₂/ZnCl₂.
   Dans un ballon de 250 mL tel que décrit à l'exemple 6, on introduit 10 g de ZnCl₂, 10 g de SnCl₂ et 5 g d'urée puis on chauffe le mélange obtenu dans un bain d'huile à 100°C pendant une durée de l'ordre de 2 heures à pression atmosphérique. On ajoute ensuite 0,1 g du catalyseur tel que décrit à l'exemple 1. On ajoute ensuite dans le ballon 5 g de méthanol, on porte la température du bain d'huile à 165°C de façon à atteindre la température de synthèse dans le ballon et on maintient cette température pendant 15 min. Toujours à la température de synthèse, on réalise une addition de méthanol à raison de 5 g de méthanol par heure pendant 4 heures en prélevant le condensat en continu. Après 4h de réaction, on interrompt l'introduction du méthanol puis, après 15 min supplémentaires, on retire le ballon du bain d'huile.
   Le taux de conversion de l'urée en carbonate de diméthyle est de 22 %.
EXEMPLE 9 - Synthèse de carbonate de diméthyle à partir d'urée, de méthanol, de chlorure d'étain (SnCℓ₂) et d'oxyde de zinc/sulfate de zinc (ZnO/ZnSO₄) à titre de catalyseur.
   Dans un réacteur de 250 mL à double enveloppe équipé d'une colonne de distillation et d'un dispositif d'agitation, on introduit 150 g de SnCℓ₂, 16 g d'urée et 0,8 g de catalyseur ZnO/ZnSO₄ tel qu'obtenu à l'exemple 1. On ajoute 25 mL de méthanol et on porte la température du fluide circulant dans la double enveloppe à la température de 165°C de façon à atteindre la température de synthèse dans le réacteur en maintenant des conditions de reflux infini pendant 15 min.
   On impose ensuite un taux de reflux de 75 (reflux/distillat) et on introduit dans le réacteur du méthanol liquide en flux continu à raison de 33,75 mL de méthanol par heure (33,75 mL/h) pendant 4 heures (soit un total de 135 mL de méthanol). Durant cette phase d'addition de méthanol, on prélève en continu un condensat en tête de la colonne de distillation. Après 4h de réaction, on interrompt l'introduction du méthanol puis, après 15 min, le chauffage du réacteur.
   L'analyse par chromatographie en phase gazeuse du condensat permet de déterminer que le taux de conversion de l'urée en carbonate de diméthyle est de 19 %. La concentration du carbonate de diméthyle dans le distillat est de 51,2 mg de carbonate de diméthyle par gramme de condensat.
EXEMPLE 10 - Synthèse de carbonate de diméthyle à partir d'urée, de méthanol, de chlorure d'étain (SnCℓ₂) et d'oxyde de zinc/sulfate de zinc (ZnO/ZnSO₄) à titre de catalyseur.
   Dans un réacteur de 250 mL à double enveloppe équipé d'une colonne de distillation et d'un dispositif d'agitation, on introduit 150 g de SnCℓ₂, 16 g d'urée et 0,8 g de catalyseur ZnO/ZnSO₄ tel qu'obtenu à l'exemple 1. On ajoute 25 mL de méthanol et on porte la température du fluide circulant dans la double enveloppe à la température de 165°C de façon à atteindre la température de synthèse dans le réacteur en maintenant des conditions de reflux infini pendant 15 min. On impose ensuite un taux de reflux de 75 (reflux/distillat) et on introduit dans le réacteur du méthanol liquide en flux continu à raison de 30 mL de méthanol par heure (30 mL/h) pendant 10,5 heures (soit un total de 315 mL de méthanol). En cours de réaction, on diminue le taux de reflux à la valeur de 20 de sorte que la température dans le réacteur dépasse 140°C. Après 10,5h de réaction, on interrompt l'introduction du méthanol puis, après 15 min, le chauffage du réacteur.
   L'analyse par chromatographie en phase gazeuse du distillat permet de déterminer que le taux de conversion de l'urée en carbonate de diméthyle est de 38,8 %. La concentration du carbonate de diméthyle dans le distillat est de 52,3 mg de carbonate de diméthyle par gramme de condensat.
EXEMPLE 11 - Enrichissement en carbonate de diméthyle d'un mélange composé de 29 % (en masse) de carbonate de diméthyle et de 71 % (en masse) de méthanol.
   Dans un ballon surmonté d'une colonne de distillation, on introduit 20 g d'un mélange composé de 29 % (en masse) de carbonate de diméthyle et de 71 % (en masse) de méthanol. On ajoute 10 g de chlorure de zinc. On chauffe le ballon de façon à former un distillat par distillation complète du mélange. La température au bouilleur en fin de distillation atteint de l'ordre de 173°C.
   On collecte ainsi 14,9 g d'un distillat comprenant 35,2 % en masse de carbonate de diméthyle correspondant à un rendement de récupération de 90,4 % du carbonate de diméthyle de départ.
EXEMPLE 12 - Enrichissement en carbonate de diméthyle d'un mélange composé de 29 % (en masse) de carbonate de diméthyle et de 71 % (en masse) de méthanol.
   Dans un ballon surmonté d'une colonne de distillation, on introduit 20 g d'un mélange composé de 29 % (en masse) de carbonate de diméthyle et de 71 % (en masse) de méthanol. On ajoute 10 g de chlorure d'étain. On chauffe le ballon de façon à collecter un distillat jusqu'à ce que la température au bouilleur atteigne 80°C environ.
   On collecte ainsi 9,3 g d'un distillat comprenant 34 % en masse de carbonate de diméthyle correspondant à un rendement de récupération de 54,5 % du carbonate de diméthyle de départ.
EXEMPLE 13 - Purification de carbonate de diméthyle d'un mélange composé de 40 % (en masse) de carbonate de diméthyle et de 60 % (en masse) de méthanol.
   Dans un bouilleur d'une capacité de 2 litres surmonté d'une colonne de distillation à 30 plateaux, on introduit 1 Kg d'un mélange composé de 40 % (en masse) de carbonate de diméthyle et de 60 % (en masse) de méthanol. On chauffe le bouilleur de façon à collecter un distillat comprenant de 35 % à 38 % de carbonate de diméthyle. En fin de distillation la température dans le bouilleur est de 93°C. On récupère dans le bouilleur 112,65 g de carbonate de diméthyle présentant une pureté de 99,8 % et représentant 28 % du carbonate de diméthyle de départ.
   L'invention peut faire l'objet de nombreuses modifications et variantes telles que par exemple le choix du dispositif de synthèse et de chauffage et les performances séparatrices de la colonne de distillation.

## Revendications

1. Procédé de synthèse de carbonate de diméthyle à partir de méthanol et d'urée, dans lequel on utilise un milieu uréique salin comprenant au moins un sel inorganique choisi dans le groupe formé du chlorure de zinc (Zn) II, des chlorures d'étain (Sn) et du chlorure de fer (Fe) III :
**caractérisé en ce que** :
- on met du méthanol, en présence d'une composition catalytique, en contact avec ledit milieu uréique salin au moins partiellement liquide à une température, dite température de synthèse, supérieure à 140°C, ce par quoi des vapeurs réactionnelles sont produites ;
- on condense les vapeurs réactionnelles et on récupère un condensat desdites vapeurs réactionnelles et comprenant du carbonate de diméthyle ;
- on met en oeuvre le procédé à pression atmosphérique.

2. Procédé selon la revendication 1, **caractérisé en ce que** lors d'une première étape opératoire, on forme un milieu réactionnel comprenant le milieu uréique salin, la composition catalytique et une première quantité de méthanol et on maintient le milieu réactionnel à la température de synthèse, le condensat formé étant réintroduit dans le milieu réactionnel, puis lors d'une deuxième étape opératoire subséquente on introduit un flux de méthanol dans le milieu réactionnel et on prélève un flux du condensat des vapeurs réactionnelles.

3. Procédé selon la revendication 2, **caractérisé en ce que** la première étape est d'une durée de l'ordre de 15 min.

4. Procédé selon l'une des revendications 2 ou 3, **caractérisé en ce qu'**on ajoute le méthanol en flux continu dans le milieu réactionnel.

5. Procédé selon l'une des revendications 2 ou 3, **caractérisé en ce qu'**on ajoute le méthanol en flux discontinu dans le milieu réactionnel.

6. Procédé selon l'une des revendications 1 à 5, **caractérisé en ce qu'**on forme initialement le milieu uréique salin par chauffage d'une composition saline comprenant chaque sel inorganique et l'urée.

7. Procédé selon la revendication 6, **caractérisé en ce qu'**on chauffe la composition saline à une température comprise entre 80 °C et 125 °C et pendant une durée d'au moins 10 min.

8. Procédé selon l'une des revendications 1 à 7, **caractérisé en ce qu'**on prépare le milieu uréique salin avec une quantité initiale d'urée et une quantité initiale de chaque sel inorganique dans un rapport massique de chaque sel inorganique par rapport à l'urée compris entre 2 et 10.

9. Procédé selon l'une des revendications 1 à 8, **caractérisé en ce que** le milieu uréique salin est un milieu conducteur.

10. Procédé selon l'une des revendications 1 à 9, **caractérisé en ce que** la composition catalytique comprend au moins un composé choisi dans le groupe formé de l'oxyde de zinc et du sulfate de zinc.

11. Procédé selon l'une des revendications 1 à 10, **caractérisé en ce qu'**on prépare la composition catalytique par calcination d'un mélange solide comprenant de l'oxyde de zinc et du sulfate de zinc à une température comprise entre 300°C et 700°C.

12. Procédé selon l'une des revendications 1 à 11, **caractérisé en ce qu'**on ajoute le méthanol à l'état liquide dans le milieu réactionnel.

13. Procédé selon l'une des revendications 1 à 12, **caractérisé en ce qu'**on introduit un flux d'au moins une partie du condensat des vapeurs réactionnelles dans un milieu réactionnel maintenu à la température de synthèse et comprenant une quantité de milieu uréique salin et une quantité de composition catalytique, ce par quoi des vapeurs réactionnelles sont produites, et on condense lesdites vapeurs réactionnelles et on récupère un condensat desdites vapeurs réactionnelles comprenant du carbonate de diméthyle.

14. Procédé selon l'une des revendications 1 à 13, **caractérisé en ce qu'**on soumet au moins une partie du condensat à une première étape de distillation fractionnée et de séparation de méthanol et d'un mélange azéotropique de carbonate de diméthyle et de méthanol.

15. Procédé selon la revendication 14, **caractérisé en ce qu'**on réalise une deuxième étape de distillation fractionnée dans laquelle on met au moins une partie du mélange azéotropique en contact avec au moins un sel inorganique et on chauffe le mélange formé de façon à former par distillation/condensation à pression atmosphérique un condensat enrichi en carbonate de diméthyle et comprenant au moins 35 % en masse de carbonate de diméthyle.

16. Procédé selon la revendication 15, **caractérisé en ce qu'**on soumet le condensat enrichi à une distillation à pression atmosphérique adaptée pour pouvoir former un condensat formé d'un mélange azéotrope de carbonate de diméthyle et de méthanol et un raffinat formé de carbonate de diméthyle.

17. Utilisation en synthèse organique d'un milieu uréique salin à l'état au moins partiellement liquide à une température supérieure à 140°C comprenant de l'urée et au moins un sel inorganique choisi dans le groupe formé du chlorure de zinc (Zn) II, des chlorures d'étain (Sn) et du chlorure de fer (Fe) III.

## Patentansprüche

1. Verfahren zur Synthese von Dimethylcarbonat aus Methanol und Harnstoff, wobei ein salzhaltiges Harnstoffmedium verwendet wird, umfassend mindestens ein anorganisches Salz, ausgewählt aus der Gruppe, gebildet aus Zink(II)chlorid (Zn), Zinnchloride (Sn) und Eisen(III)chlorid (Fe):
**dadurch gekennzeichnet, dass**:
- Methanol, in Anwesenheit einer katalytischen Zusammensetzung, mit dem mindestens teilweise flüssigen salzhaltigen Harnstoffmedium bei einer Temperatur, bezeichnet als Synthesetemperatur, von mehr als 140 °C in Kontakt gebracht wird, wodurch Reaktionsdämpfe erzeugt werden;
- die Reaktionsdämpfe kondensiert und ein Kondensat der Reaktionsdämpfe und umfassend Dimethylcarbonat, wiedergewonnen wird;
- das Verfahren bei atmosphärischem Druck durchgeführt wird.

2. Verfahren nach Anspruch 1, **dadurch gekennzeichnet, dass** beim ersten Betriebsschritt ein Reaktionsmedium gebildet wird, umfassend das salzhaltige Harnstoffmedium, die katalytische Zusammensetzung und eine erste Menge Methanol, und das Reaktionsmedium bei einer Synthesetemperatur gehalten wird, wobei das gebildete Kondensat wieder in das Reaktionsmedium eingeführt wird, dann während eines folgenden zweiten Betriebsschritts ein Methanolstrom in das Reaktionsmedium eingeführt wird und ein Strom des Kondensats den n entnommen wird.

3. Verfahren nach Anspruch 2, **dadurch gekennzeichnet, dass** der erste Schritt eine Dauer im Bereich von 15 min. aufweist.

4. Verfahren nach einem der Ansprüche 2 oder 3, **dadurch gekennzeichnet, dass** das Methanol in ununterbrochenem Strom in das Reaktionsmedium zugegeben wird.

5. Verfahren nach einem der Ansprüche 2 oder 3, **dadurch gekennzeichnet, dass** das Methanol in unterbrochenem Strom in das Reaktionsmedium zugegeben wird.

6. Verfahren nach einem der Ansprüche 1 bis 5, **dadurch gekennzeichnet, dass** anfänglich das salzhaltige Harnstoffmedium durch Erhitzung einer salzhaltigen Zusammensetzung gebildet wird, umfassend jedes anorganische Salz und Harnstoff.

7. Verfahren nach Anspruch 6, **dadurch gekennzeichnet, dass** die salzhaltige Zusammensetzung auf eine Temperatur, die zwischen 80 °C und 125 °C liegt, und während einer Dauer von mindestens 10 min. erhitzt wird.

8. Verfahren nach einem der Ansprüche 1 bis 7, **dadurch gekennzeichnet, dass** das salzhaltige Harnstoffmedium mit einer anfänglichen Menge von Harnstoff und einer anfänglichen Menge jedes anorganischen Salzes in einem Mengenverhältnis jedes anorganischen Salzes im Verhältnis zum Harnstoff, das zwischen 2 und 10 liegt, hergestellt wird.

9. Verfahren nach einem der Ansprüche 1 bis 8, **dadurch gekennzeichnet, dass** das salzhaltige Harnstoffmedium ein leitendes Medium ist.

10. Verfahren nach einem der Ansprüche 1 bis 9, **dadurch gekennzeichnet, dass** die katalytische Zusammensetzung mindestens eine Verbindung umfasst, ausgewählt aus der Gruppe, gebildet aus Zinkoxid und Zinksulfat.

11. Verfahren nach einem der Ansprüche 1 bis 10, **dadurch gekennzeichnet, dass** die katalytische Zusammensetzung durch Kalzinierung einer stabilen Mischung hergestellt wird, umfassend Zinkoxid und Zinksulfat bei einer Temperatur, die zwischen 300 °C und 700 °C liegt.

12. Verfahren nach einem der Ansprüche 1 bis 11, **dadurch gekennzeichnet, dass** das Methanol in flüssigem Zustand in das Reaktionsmedium zugegeben wird.

13. Verfahren nach einem der Ansprüche 1 bis 12, **dadurch gekennzeichnet, dass** ein Strom von mindestens einem Teil des Kondensats der Reaktionsdämpfe in das Reaktionsmedium eingeführt wird, das bei einer Synthesetemperatur gehalten wird und umfassend eine Menge salzhaltiges Harnstoffmedium und eine Menge katalytischer Zusammensetzung, wodurch Reaktionsdämpfe erzeugt werden; und die Reaktionsdämpfe kondensiert und ein Kondensat der Reaktionsdämpfe umfassend Dimethylcarbonat, wiedergewonnen wird.

14. Verfahren nach einem der Ansprüche 1 bis 13, **dadurch gekennzeichnet, dass** mindestens ein Teil des Kondensats einem ersten Schritt des fraktionierten Destillierens und des Trennens des Methanols und einer azeotropen Mischung aus Dimethylcarbonat und Methanol unterzogen wird.

15. Verfahren nach Anspruch 14, **dadurch gekennzeichnet, dass** ein zweiter Schritt des fraktionierten Destillierens, in dem mindestens ein Teil der azeotropen Mischung mit mindestens einem anorganischen Salz in Kontakt gebracht wird und die gebildete Mischung erhitzt wird, um durch Destillation/Kondensation bei atmosphärischem Druck ein Kondensat zu bilden, das mit Dimethylcarbonat angereichert ist und mindestens 35 Massenprozent Dimethylcarbonat umfasst.

16. Verfahren nach Anspruch 15, **dadurch gekennzeichnet, dass** das angereicherte Kondensat einer Destillation bei atmosphärischem Druck unterzogen wird, die ausgelegt ist, um ein Kondensat bilden zu können, das aus einer azeotropen Mischung aus Dimethylcarbonat und Methanol und einem Raffinat, gebildet aus Dimethylcarbonat, gebildet ist.

17. Verwendung bei der organischen Synthese eines salzhaltigen Harnstoffmediums in mindestens teilweise flüssigem Zustand bei einer Temperatur von mehr als 140 °C, umfassend Harnstoff und mindestens ein anorganisches Salz, ausgewählt aus der Gruppe, gebildet aus Zink(II)chlorid (Zn), Zinnchloriden (Sn) und Eisen(III)chlorid (Fe).

## Claims

1. Method for synthesizing dimethyl carbonate starting from methanol and urea, in which a saline ureic medium comprising at least one inorganic salt selected from the group formed by zinc chloride (Zn) II, tin chlorides (Sn), and iron chloride (Fe) III is used:
**characterised in that**:
- Methanol, in the presence of a catalytic composition, is brought into contact with said saline ureic medium that is at least partially liquid at a temperature, so-called synthesis temperature, that is higher than 140°C, such that reaction vapors are produced;
- The reaction vapors are condensed, and a condensate of said reaction vapors and that comprises dimethyl carbonate is recovered;
- The method is implemented at atmospheric pressure.

2. Method according to Claim 1, **characterised in that** during a first operating step, a reaction medium that comprises the saline ureic medium, the catalytic composition, and a first quantity of methanol is formed, and the reaction medium is kept at the synthesis temperature, with the formed condensate being reintroduced into the reaction medium, and then during a second subsequent operating step, a stream of methanol is introduced into the reaction medium, and a stream of the condensate of the reaction vapors is sampled.

3. Method according to Claim 2, **characterised in that** the first step is of a duration of about 15 minutes.

4. Method according to one of Claims 2 or 3, **characterised in that** methanol is added in a continuous stream into the reaction medium.

5. Method according to one of Claims 2 or 3, **characterised in that** methanol is added in an intermittent stream into the reaction medium.

6. Method according to one of Claims 1 to 5, **characterised in that** the saline ureic medium is formed initially by heating a saline composition comprising each inorganic salt and urea.

7. Method according to Claim 6, **characterised in that** the saline composition is heated at a temperature of between 80°C and 125°C and for a duration of at least 10 minutes.

8. Method according to one of Claims 1 to 7, **characterised in that** the saline ureic medium is prepared with an initial quantity of urea and an initial quantity of each inorganic salt in a ratio by mass of each inorganic salt in relation to the urea of between 2 and 10.

9. Method according to one of Claims 1 to 8, **characterised in that** the saline ureic medium is a conductive medium.

10. Method according to one of Claims 1 to 9, **characterised in that** the catalytic composition comprises at least one compound that is selected from the group formed by zinc oxide and zinc sulfate.

11. Method according to one of Claims 1 to 10, **characterised in that** the catalytic composition is prepared by calcination of a solid mixture comprising zinc oxide and zinc sulfate at a temperature of between 300°C and 700°C.

12. Method according to one of Claims 1 to 11, **characterised in that** methanol is added in the liquid state into the reaction medium.

13. Method according to one of Claims 1 to 12, **characterised in that** a stream of at least one portion of the condensate of reaction vapors is introduced into a reaction medium kept at the synthesis temperature and comprising a quantity of saline ureic medium and a quantity of catalytic composition, such that reaction vapors are produced, and said reaction vapors are condensed, and a condensate of said reaction vapors comprising dimethyl carbonate is recovered.

14. Method according to one of Claims 1 to 13, **characterised in that** at least a portion of the condensate is subjected to a first step for fractionated distillation and for separation of methanol and an azeotropic mixture of dimethyl carbonate and methanol.

15. Method according to Claim 14, **characterised in that** a second fractionated distillation step is carried out in which at least a portion of the azeotropic mixture is brought into contact with at least one inorganic salt, and the mixture that is formed is heated in such a way as to form - by distillation/condensation at atmospheric pressure - a condensate that is enriched with dimethyl carbonate and that comprises at least 35% by mass of dimethyl carbonate.

16. Method according to Claim 15, **characterised in that** the enriched condensate is subjected to a distillation at atmospheric pressure that is suitable for being able to form a condensate that is formed by an azeotropic mixture of dimethyl carbonate and methanol and a raffinate formed by dimethyl carbonate.

17. Use in organic synthesis of a saline ureic medium in the at least partially liquid state at a temperature of higher than 140°C comprising urea and at least one inorganic salt that is selected from the group that is formed by zinc chloride (Zn) II, tin chlorides (Sn), and iron chloride (Fe) III.
